# DEMANDE DE BREVET EUROPEEN

(11) **EP 1 911 420 A1**
(43) Date de publication de la demande: **16.04.2008**
(21) Numéro de dépôt: 07356136.7
(22) Date de dépôt: 12.10.2007
(51) Int. Cl.: A61F 2/42

(54) **Ensemble prothetique de cheville**

(30) Priorité: 13.10.2006 FR 0609001
(71) Demandeur: TORNIER SA, 38330 Saint Ismier (FR)
(72) Inventeur: Ferrari, Irène, 38660 Le Touvet (FR); Weber, Michael, 52074 Aachen (DE)
(74) Mandataire: Grand, Guillaume

(57) **Abrégé**

Cet ensemble comporte, d'une part, un composant astragalien (20), à fixer sur l'astragale (A) d'un patient et muni, par exemple, d'un taraudage interne (22A) débouchant sur le côté inférieur (21B) du composant astragalien, et, d'autre part, une tige astragalo-calcanéenne (40) munie, à sa première extrémité longitudinale (41), d'un filetage (41A) complémentaire du taraudage. Pour traiter, en une seule intervention chirurgicale, une cheville dont on veut fusionner l'astragale et le calcanéum (C) tout en rétablissant l'articulation entre l'astragale et le tibia, la tige astragalo-calcanéenne est munie, à sa seconde extrémité longitudinale (43), d'un moyen de mise en prise osseuse du calcanéum, tel qu'un filetage (43A) à engager dans la matière osseuse du calcanéum ou un anneau à appuyer contre une face inférieure du calcanéum, pour rapprocher et ainsi comprimer le calcanéum contre l'astragale lors du vissage de la première extrémité de la tige dans le taraudage du composant astragalien.

## Description

La présente invention concerne un ensemble prothétique de cheville, ainsi qu'une méthode chirurgicale de pose d'un tel ensemble.

L'invention s'intéresse plus particulièrement au traitement d'une cheville dont l'articulation entre le tibia et l'astragale et l'articulation entre l'astragale et le calcanéum sont altérées.

Une possibilité de traitement consiste à fusionner les trois os du calcanéum, de l'astragale et du tibia, comme proposé dans US-A-2005/0107791. Une longue vis de compression relie par l'intérieur le tibia et le calcanéum, en passant par l'astragale, de manière à bloquer ces trois os les uns par rapport aux autres. Il en résulte une perte de mobilité importante pour le patient, puisque sa cheville est totalement bloquée.

Une autre possibilité de traitement consiste, d'une part, à fusionner les os du calcanéum et de l'astragale, par le biais d'une tige d'arthrodèse dédiée et, d'autre part, à rétablir les capacités articulaires entre les os de l'astragale et du tibia, en posant une prothèse articulaire de cheville. Cependant, les deux articulations doivent alors être traitées en deux temps : l'arthrodèse du calcanéum et de l'astragale nécessite une consolidation d'au moins six mois, ce qui reporte d'autant la pose de la prothèse articulaire entre l'astragale et le tibia.

Par ailleurs, on connaît par US-A-2005/0288792, un composant astragalien d'une prothèse articulaire de cheville, permettant de rétablir des capacités articulaires entre l'astragale et le tibia, tout en étant associé à une tige astragalo-calcanéenne s'étendant depuis un côté inférieur du composant astragalien jusqu'à la partie supérieure du calcanéum, en traversant l'astragale de part en part. Dans un mode de réalisation, l'extrémité supérieure de cette tige est directement vissée dans un taraudage interne du composant astragalien, qui débouche sur le côté inférieur de ce composant. Avant d'être implanté, le composant et la tige sont fermement immobilisés l'un par rapport à l'autre, par vissage. Lorsque cet ensemble vissé est implanté, la tige garantit un bon ancrage du composant astragalien puisque ce dernier est ainsi fixé, à la fois, à l'astragale et à la partie supérieure du calcanéum, ce qui explique que cet ensemble prothétique est plus spécifiquement destiné à la révision d'une arthroplasie de la cheville. Cependant, cet ensemble ne bloque pas de manière efficace l'articulation entre le calcanéum et l'astragale, puisque des débattements relatifs autour et/ou le long de la tige restent possibles et, en pratique, se produisent rapidement.

FR-A-2 220 235 propose un ensemble prothétique de cheville, comportant un composant astragalien d'une prothèse articulaire de cheville, sous la forme d'un support adapté pour être fixé sur l'astragale d'un patient. Ce support est destiné à coopérer de manière articulée avec une tige tibiale, avec interposition d'une pièce intermédiaire articulée. Dans sa partie inférieure, le support astragalien est associé à trois pieds d'ancrage, à savoir deux pieds antérieurs et un pied postérieur, respectivement destinés à être encastrés dans des cavités correspondantes, réalisées dans l'astragale et dans le calcanéum. Pour ce qui concerne le pied postérieur, un mode de réalisation prévoit que ce pied est constitué d'au moins une vis sagittale qui traverse le support de haut en bas, jusqu'à prendre appui par sa tête sur ce support, tandis que sa tige est enfichée, sans être vissée, dans la cavité du calcanéum, bourrée de ciment. L'intérêt de recourir à une telle vis est que, si besoin, elle peut être dévissée pour faciliter l'enlèvement du support. Ainsi, le pied postérieur de cet ensemble prothétique permet de former un appui stable sur le calcanéum mais, même sous la forme d'une vis enfichée dans du ciment, ce pied ne bloque pas totalement l'articulation entre l'astragale et le calcanéum de manière efficace.

WO-A-01/30264 et US-A-2005/124995 proposent, quant à eux, un ensemble prothétique de cheville, comportant une tige astagalo-calcanéenne introduite dans une cavité préalablement réalisée à travers l'astragale et dans la partie supérieure du calcanéum. Cette tige sert d'ancrage à une plaque astragalienne, qui est mise en place et fixée à la tige astragalo-calcanéenne après que cette tige ait été mise en place dans la cavité osseuse précitée. Aucun blocage efficace de l'articulation entre le calcanéum et l'astragale n'est donc obtenu par cette tige, si bien que pour limiter les débattements relatifs entre ces deux os, des vis additionnelles sont rapportées entre la plaque astragalienne et la tige astragolo-calcanéenne, afin de stabiliser la position de cette dernière.

Le but de la présente invention est de proposer un ensemble prothétique de cheville qui, tout en pouvant être implanté en une seule intervention, permet, à la fois, de bloquer l'articulation entre le calcanéum et l'astragale et de rétablir une capacité articulaire entre l'astragale et le tibia d'un patient.

A cet effet, l'invention a pour objet un ensemble prothétique de cheville, tel que défini à la revendication 1.

Lorsque l'ensemble prothétique selon l'invention est implanté, son composant astragalien permet des mouvements articulaires entre l'astragale et le tibia d'un patient, notamment en étant associé à un composant prothétique tibial fixé sur le tibia et à un patin prothétique interposé entre les composants astragalien et tibial, tandis que la tige astragalo-calcanéenne bloque l'articulation entre le calcanéum et l'astragale, par compression de ces deux os l'un contre l'autre. En pratique, cette compression osseuse est obtenue par la fixation de la première extrémité de la tige dans le composant astragalien, ce qui met en prise la seconde extrémité de la tige directement avec le calcanéum. A cet effet, un moyen de fixation et le moyen de mise en prise respectivement prévus aux extrémités de la tige sont structurellement prévus pour obtenir un effet de compression progressif entre le calcanéum et l'astragale au fur et à mesure que la seconde extrémité de la tige progresse dans le calcanéum alors que la première extrémité est accouplée à la partie dédiée du composant astragalien appuyé sur l'astragale.

En pratique, la tige est introduite depuis une face inférieure du calcanéum, dans un canal astragalo-calcanéen qui a été préalablement creusé à travers le calcanéum et l'astragale et qui débouche sur la partie dédiée du composant astragalien lorsque ce dernier est placé sur une face supérieure de l'astragale. Ainsi, avantageusement en une seule intervention chirurgicale, le composant astragalien et la tige astragalo-calcanéenne sont implantés, en étant accouplés l'un à l'autre au cours de l'intervention, alors que la tige traverse de part en part le calcanéum et l'astragale, afin de les comprimer et ainsi les immobiliser l'un contre l'autre. Autrement dit, l'opération d'artroplasie entre l'astragale et le tibia et l'opération d'arthrodèse entre l'astragale et le calcanéum sont réalisées au cours de la même intervention chirurgicale, ce qui limite les risques opératoires pour le patient et ce qui permet au chirurgien d'utiliser globalement les mêmes passages chirurgicaux pour les deux opérations.

En outre, la tige astragalo-calcanéenne assure l'immobilisation du composant astragalien par rapport à l'astragale et au calcanéum, ce qui améliore la tenue du composant astragalien. De plus, en cas de révision de l'ensemble prothétique selon l'invention, l'appui sur le calcanéum est sûr et fiable, dans le sens où cet os est fermement fusionné avec l'astragale.

D'autres caractéristiques avantageuses de cet ensemble prothétique de cheville, prises isolément ou suivant toutes les combinaisons techniquement possibles, sont énoncées aux revendications 2 à 11.

L'invention a également pour objet une méthode chirurgicale de pose d'un ensemble prothétique de cheville, ledit ensemble comprenant un composant astragalien d'une prothèse articulaire de cheville une tige astragalo-calcanéenne dont une première de ses deux extrémités longitudinales est adaptée pour être fixée au composant astragalien, par complémentarité de formes avec une partie dédiée de ce composant, et dont la seconde extrémité longitudinale est munie d'un moyen de mise en prise osseuse, cette méthode de pose comprenant des étapes peropératoires suivant lesquelles :
i) on prépare une face supérieure de l'astragale d'un patient pour fixer le composant astragalien,
ii) on perce conjointement l'astragale et le calcanéum de part en part, entre la face supérieure de l'astragale et une face inférieure du calcanéum opposée à l'astragale, de manière à former un canal astragalo-calcanéen,
iii) on place le composant astragalien sur la face supérieure de l'astragale, le canal astragalo-calcanéen débouchant alors sur la partie dédiée du composant astragalien,
iv) on insère la tige astragalo-calcanéenne dans le canal astragalo-calcanéen depuis la face inférieure du calcanéum, et
v) on fixe la première extrémité de la tige astragale-calconéenne dans la partie dédiée du composant astragalien, de manière que le moyen de mise en prise prévu à la seconde extrémité de la tige rapproche et ainsi comprime le calcanéum contre l'astragale.

Cette méthode permet, en une seule intervention chirurgicale, de poser un ensemble prothétique de cheville tel que défini ci-dessus, de manière à permettre, conjointement, l'arthrodèse du calcanéum et de l'astragale et l'artroplasie entre l'astragale et le tibia d'un patient.

Suivant une mise en oeuvre avantageuse de cette méthode, lors de l'étape i), on creuse dans la face supérieure de l'astragale un évidement sensiblement cylindrique de réception d'un plot creux d'ancrage osseux dont est muni le composant astragalien et dans lequel est agencée la partie dédiée, et lors de l'étape ii), on perce depuis la face supérieure de l'astragale le canal astragalo-calcanéen dans un prolongement de l'évidement.

Dans ce cas, pour plus de praticité, on utilise un canon de perçage pour percer le canal astragalo-calcanéen, en faisant prendre appui ce canon dans l'évidement creusé lors de l'étape i).

Avantageusement, pour favoriser la fusion du calcanéum et de l'astragale, la méthode comprend, avant l'étape ii), une étape peropératoire additionnelle lors de laquelle on introduit une matière osseuse entre l'astragale et le calcanéum par athroscopie.

Par ailleurs, après consolidation de l'arthrodèse du calcanéum et de l'astragale, la méthode comprend avantageusement une étape postopératoire suivant laquelle on retire la tige astragalo-calcanéenne en désaccouplant sa première extrémité de la partie dédiée du composant astragalien.

L'invention sera mieux comprise à la lecture de la description qui va suivre, donnée uniquement à titre d'exemple et faite en se référant aux dessins sur lesquels :
- la figure 1 est une vue frontale de devant, avec coupe partielle, d'un ensemble prothétique de cheville selon l'invention, implanté au niveau de la cheville gauche d'un patient ;
- la figure 2 est une vue éclatée en élévation latérale, selon la flèche Il indiquée à la figure 1, d'une partie de l'ensemble prothétique, représentée sans les os de la cheville ;
- les figures 3A et 3B sont des coupes schématiques selon la ligne III-III de la figure 1, illustrant respectivement deux étapes successives de la pose de l'ensemble prothétique ; et
- la figure 4 est une vue analogue à la figure 3B, illustrant une variante de l'ensemble prothétique selon l'invention.

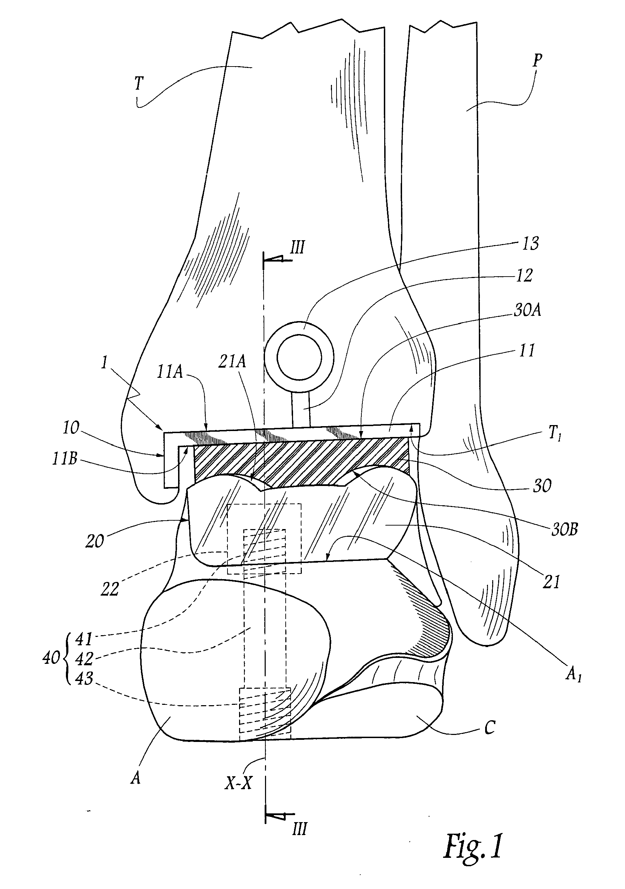

Sur la figure 1 sont représentés, de manière schématique, les extrémités inférieures d'un tibia T et d'un péroné P d'un être humain, ainsi que l'astragale A et le calcanéum C correspondants, illustrant ainsi les constituants osseux de la cheville gauche d'un patient. Sur la figure 1 est également représenté un ensemble prothétique de cheville 1, comportant quatre composants distincts implantés au niveau de la cheville du patient, à savoir un composant tibial 10, un composant astragalien 20, un patin prothétique 30, interposé entre les composants tibial et astragalien, et une tige astragalo-calcanéenne 40. A la figure 1, seul le patin 30 est représenté en coupe frontale, tandis que les traits pointillés sont utilisés pour figurer les contours des composants masqués par de la matière osseuse.

Par commodité, la suite de la description est orientée par rapport aux os de la cheville dans leur position anatomique, c'est-à-dire que les termes « postérieur » ou « arrière », « antérieur » ou « avant », « supérieur », « inférieur », etc. s'entendent par rapport à la cheville du patient se tenant debout sur une surface sensiblement horizontale.

Le composant tibial 10 comporte une plaque 11 rapportée de manière fixe à l'extrémité inférieure du tibia T. A cet effet, la plaque 11 est, sur son côté supérieur 11A, venue de matière, par une aile sagittale 12, avec un plot creux d'ancrage osseux 13. Des moyens d'ancrage de la plaque 11 additionnels et/ou alternatifs sont envisageables, du moment qu'ils immobilisent efficacement le composant tibial à l'extrémité inférieure du tibia T.

Sur son côté inférieur, la plaque 11 délimite une surface plane 11 B destinée à former un appui glissant pour la surface supérieure 30A plane du patin 30.

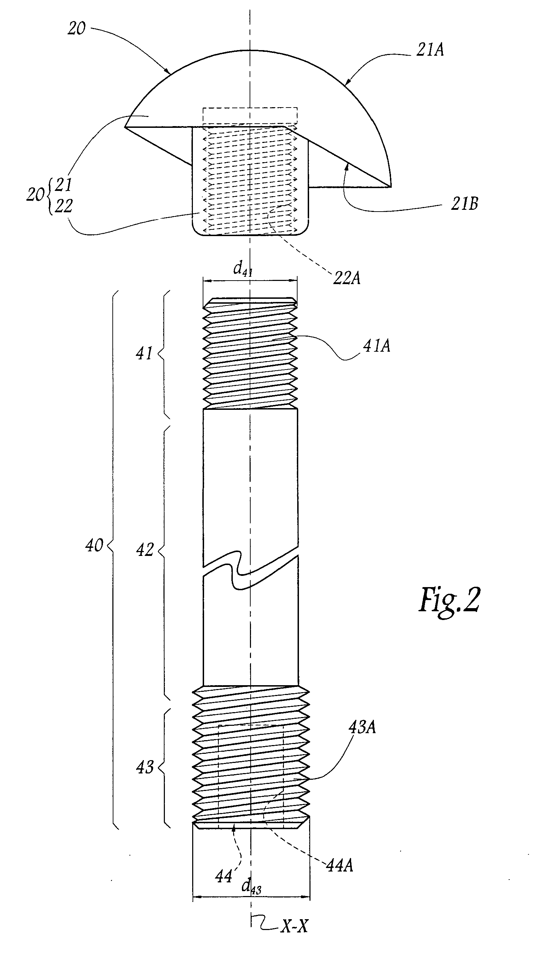

Le composant astragalien 20 comporte un bloc principal 21 rapporté de manière fixe à l'extrémité supérieure de l'astragale A. A cet effet, le bloc 21 est venu de matière avec un plot creux d'ancrage osseux 22 s'étendant vers le bas depuis le côté inférieur 21 B du bloc 21, comme représenté en pointillés à la figure 1 et comme bien visible à la figure 2.

Sur son côté supérieur, le bloc 21 délimite une surface articulaire 21A destinée à coopérer avec une surface articulaire conjuguée 30B délimitée par le côté inférieur du patin 30. En coupe sagittale, la surface 21A présente un profil arqué de concavité tournée vers le bas, comme visible à la figure 2. Les surfaces articulaires 21A et 30B sont ainsi adaptées pour glisser l'une contre l'autre le long de ce profil incurvé, dans une direction globalement antéro-postérieure. Avantageusement, ces surfaces 21A et 30B admettent entre elles un débattement médio-latéral, qui se combine au mouvement incurvé de glissement dans la direction antéro-postérieure.

Comme représenté à la figure 2, la tige 40 s'étend en longueur autour d'un axe central X-X globalement vertical. Successivement suivant sa longueur, cette tige comporte une première extrémité filetée 41, une partie courante 42 extérieurement lisse et une seconde extrémité filetée 43. L'extrémité 41, la partie courante 42 et l'extrémité 43 sont successivement étagées de manière croissante, de sorte que, d'une part, le diamètre maximal d₄₁ du filetage 41A de l'extrémité 41, c'est-à-dire la dimension transversale maximale de cette extrémité 41 au niveau de la crête du filetage, est inférieur ou égal au diamètre extérieur de la partie courante 42 et, d'autre part, le diamètre maximal d₄₃ du filetage 43A de l'extrémité 43, c'est-à-dire la dimension transversale maximale de cette extrémité 43 au niveau de la crête du filetage 43A, est strictement supérieur au diamètre de la partie courante 42.

Le filetage 41A de l'extrémité 41 est adapté pour être reçu par vissage dans un taraudage complémentaire 22A qui, comme indiqué en pointillés à la figure 2, est délimité par la face intérieure du plot creux 22 en étant de préférence centré sur l'axe longitudinal central de ce plot.

Au niveau de son extrémité 43, la tige 40 délimite un évidement 44 sensiblement centré sur l'axe X-X et débouchant axialement sur l'extérieur vers le bas, comme indiqué en pointillés à la figure 2. Cet évidement 44 présente un profil transversal 44A, par exemple de forme carrée, destiné à coopérer avec un outil complémentaire en vue d'entraîner la tige 40 en rotation sur elle-même autour de l'axe X-X.

On va décrire ci-après une méthode chirurgicale visant à implanter l'ensemble prothétique de cheville 1.

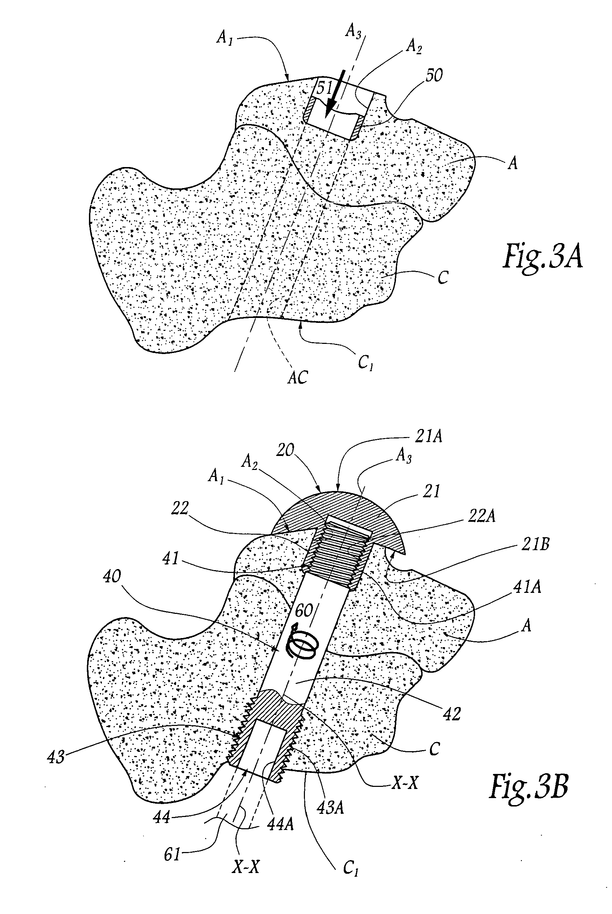

Dans un premier temps, les os de la cheville du patient doivent être préparés. A cet effet, le chirurgien utilise des moyens de coupe non représentés de manière à, d'une part, réséquer l'extrémité inférieure du tibia T pour former une surface T₁ sensiblement plane et, d'autre part, réséquer l'extrémité supérieure de l'astragale A pour former une face multi-plans A₁ comportant deux surfaces principales planes inclinées l'une par rapport à l'autre, comme visibles à la figure 3A. Dans la face A₁, un évidement cylindrique A₂, représenté en traits pleins à la figure 3A, est creusé suivant une direction globalement verticale, par exemple au moyen d'une fraise. Cet évidement A₂ définit ainsi un axe longitudinal central A₃.

A ce stade de l'intervention chirurgicale, la face supérieure A₁ de l'astragale A est dans une configuration convenable pour recevoir le composant astragalien 20, étant entendu que l'évidement A₂ est prévu sensiblement complémentaire du plot 22 pour ancrer ce composant 20 sur l'astragale. Cependant, avant de mettre en place le composant astragalien 20, le chirurgien perce l'astragale A et le calcanéum C de part en part, depuis la face supérieure A₁ de l'astragale jusqu'à une face inférieure C₁ du calcanéum, de manière à former un canal astragalo-calcanéen AC indiqué en pointillés à la figure 3A. Ce canal AC est creusé dans un prolongement rectiligne de l'évidement A₂, en étant sensiblement centré sur l'axe A₃. Pour ce faire, le chirurgien utilise avantageusement un canon de perçage 50 représenté de manière partielle et schématique à la figure 3A : ce canon 50 présente une forme tubulaire adaptée pour être reçu de manière sensiblement complémentaire dans l'évidement A₂, de façon sensiblement co-axiale à cet évidement. De la sorte, le canon 50 prend appui dans l'évidement A₂, de manière à guider l'introduction et la progression d'une mèche de perçage, successivement à travers l'astragale et le calcanéum, comme indiqué par la flèche 51 à la figure 3A. Le chirurgien perce ainsi le canal astragalo-calcanéen AC de manière rapide et précise, en utilisant des ancillaires traditionnels dans le domaine de la chirurgie orthopédique.

Après le perçage du canal astragalo-calcanéen AC, le chirurgien met en place, à la fois, le composant astragalien 20 sur la face supérieure A₁ de l'astragale A et la tige astragalo-calcanéenne 40 dans le canal AC. Plus précisément, la tige 40 est introduite dans le canal AC depuis la face inférieure C₁ du calcanéum, avec son extrémité 41 dirigée vers le haut. Le diamètre interne du canal AC étant prévu sensiblement égal au diamètre extérieur de la partie courante 42, la progression de la tige dans le canal s'effectue aisément, selon un mouvement de translation vers le haut et parallèlement à l'axe A₃, jusqu'à ce que l'extrémité 41 atteigne le débouché du canal AC dans l'évidement A₂. Cet évidement étant occupé par le plot 22, la progression de la tige 40 nécessite l'entraînement de cette dernière en rotation sur elle-même autour de l'axe X-X, de manière à visser le filetage 41A dans le taraudage 22A, comme indiqué par la flèche 60. En pratique, l'entraînement rotatif de la tige est réalisé au moyen d'un outil, dont la tête, indiquée uniquement en pointillés par la référence 61 à la figure 3B, est mise en prise avec le profil 44A dans l'évidement 44.

On comprend que, par un dimensionnement en longueur adéquat de la tige 40, le vissage de l'extrémité 41 dans le plot 22 s'accompagne du vissage de l'extrémité 43 dans le calcanéum, le filetage 43A s'engageant directement dans la matière osseuse du calcanéum, au niveau du débouché du canal AC sur la face inférieure C₁ du calcanéum. Autrement dit, la tige 40 présente une longueur sensiblement égale à celle du canal astragalo-calcanéen AC, de manière que la tige puisse s'étendre à travers l'astragale et le calcanéum avec son extrémité 41 au niveau de la face supérieure A₁ de l'astragale et avec son extrémité 43 au niveau de la face inférieure C₁ du calcanéum.

Le double vissage des filetages 41A et 43A provoque le rapprochement progressif l'un vers l'autre de l'astragale A et du calcanéum C, mettant ainsi en compression ces deux os l'un contre l'autre, au niveau de leur face respective en contact. On comprend que les caractéristiques structurelles de ces filetages, notamment leur pas respectif, sont choisis pour atteindre des valeurs de compression suffisantes pour bloquer efficacement l'astragale et le calcanéum l'un contre l'autre, sans engendrer de contrainte osseuse excessive.

Parallèlement, les autres composants de l'ensemble prothétique 1 sont implantés : le composant tibial 10 est rapporté et immobilisé à l'extrémité inférieure du tibia T, avec sa plaque 11 en appui plan contre la face T₁, puis le patin prothétique 30 est rapporté entre les composants 10 et 20. L'ensemble 1 est alors dans la configuration d'implantation illustré à la figure 1 et l'intervention chirurgicale prend fin.

Après consolidation de l'arthrodèse de l'astragale A et du calcanéum C, qui prend généralement entre six et douze mois, la tige astragalo-calcanéenne 40 peut être retirée, par dévissage de ses extrémités 41 et 43. Le chirurgien utilise alors avantageusement le profil 44A pour y introduire un outil de dévissage approprié. Pour favoriser et accélérer cette arthrodèse, de l'os peut avantageusement être introduit entre l'astragale et le calcanéum avant la mise en place de l'ensemble prothétique 1 : avant de percer conjointement l'astragale A et le calcanéum C de part en part, on utilise un arthroscope débouchant dans la zone de jonction entre l'astragale et le calcanéum, pour y placer de la matière osseuse.

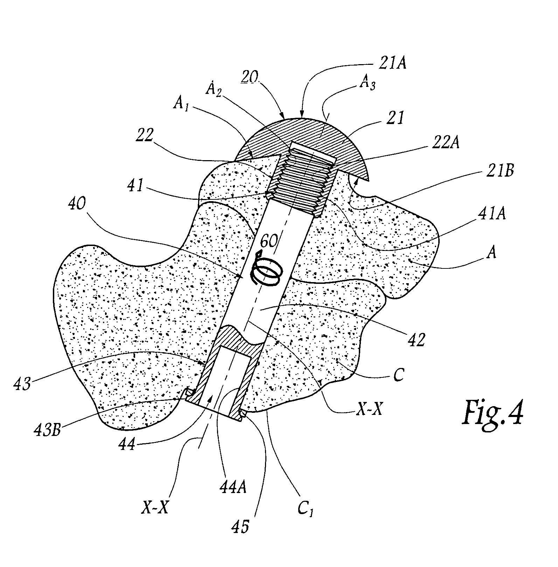

Sur la figure 4 est représentée une variante de la tige astragalo-calcanéenne 40, qui ne se distingue du mode de réalisation considéré aux figures 1 à 3B qu'au niveau de son extrémité inférieure 43 : le filetage 43A est supprimé, si bien que la surface extérieure de l'extrémité 43 est lisse et s'étend dans le prolongement rectiligne de la surface extérieure de la partie courante 42, excepté au niveau de sa zone terminale qui forme un renflement 43B radialement saillant vers l'extérieur depuis le reste de cette surface. A la différence du mode de réalisation des figures 1 à 3B, l'extrémité 43 de la tige 40 est, pour le mode de réalisation de la figure 4, associé à un anneau 45 entourant extérieurement cette extrémité. Cet anneau présente un diamètre intérieur qui, à la fois, est sensiblement égal ou supérieur au diamètre extérieur de l'extrémité 43 excepté au niveau de son renflement terminal 43B, et est inférieur au diamètre extérieur maximal de ce renflement. En pratique, l'anneau 45 est enfilé autour de la tige 40 en étant introduit depuis l'extrémité supérieure 41 alors que la tige n'est pas encore introduite dans le canal astragalo-calcanéen AC.

On comprend que, lorsque le filetage 41A de l'extrémité 41 de la tige 40 est vissé dans le taraudage 22A du composant astragalien 20, par entraînement rotatif de la tige au moyen de l'outil dont la tête 61 est mise en prise dans l'évidement 44, l'anneau 45 se trouve interposé entre la face inférieure C₁ du calcanéum C et le renflement terminal 43B de la tige, jusqu'à ce que ce renflement entraîne l'anneau 45 en appui pressant contre la face C₁ du calcanéum. L'anneau 45 transmet et répartit ainsi l'effort de serrage sur la face C₁ du calcanéum, à la façon d'une rondelle. Le calcanéum se trouve alors comprimé contre l'astragale A, jusqu'à bloquer efficacement l'astragale et le calcanéum l'un contre l'autre.

L'existence d'un jeu radial entre la face intérieure de l'anneau 45 et la face extérieure de l'extrémité 43, au dessus de son renflement terminal 43B, permet l'ajustement de la position de l'anneau par rapport à la tige lors de son appui pressant contre le calcanéum C. Cette mobilité relative entre l'anneau et l'extrémité 43 compense ainsi l'éventuel désalignement entre l'axe central de l'anneau et l'axe X-X de la tige.

En variante non représentée, l'anneau 45 est solidarisé fixement à l'extrémité inférieure 43 de la tige 40, en étant par exemple directement venu de matière avec la tige. Dans ce cas, pour augmenter la surface de la zone d'appui de cet anneau fixe contre la face inférieure C₁ du calcanéum C lors du vissage du filetage 41A dans le composant astragalien 20, cette face calcanéenne C₁ est, si besoin, préalablement travaillée de manière à délimiter, autour du débouché du canal astragalo-calcanéen AC, une surface sensiblement plane, inscrite dans un plan essentiellement perpendiculaire à l'axe A₃ de ce canal.

D'autres formes de réalisation que le filetage 43A et l'anneau 45 peuvent être prévues à l'extrémité inférieure 43 de la tige astragalo-calcanéenne 40 pour mettre en prise le calcanéum C et le comprimer contre l'astragale A lors du vissage de l'extrémité supérieure 41 dans le composant astragalien 20 : l'extrémité inférieure 43 peut par exemple présenter une surface extérieure en forme de tronc de cône ou de pavillon, divergent vers le bas.

Divers autres aménagements et variantes à l'ensemble prothétique de cheville 1 et à la méthode de pose décrits ci-dessus sont par ailleurs envisageables. A titre d'exemples :
- l'axe central du taraudage 22A peut être décalé de l'axe central du plot 22, le canon de perçage 50 étant alors prévu avec un décalage analogue pour percer le canal astragalo-calcanéen AC dans le prolongement, éventuellement incliné, de l'évidemment A₂ ;
- la structure taraudage 22A/filetage 41A peut être inversée, de sorte que l'extrémité 41 de la tige 40 forme un taraudage à même d'être vissé autour d'un filetage agencé dans le plot 22, par exemple délimité par un téton central interne à ce plot ; de manière plus générale, d'autres formes de moyens permettant l'accouplement par complémentarité de formes entre l'extrémité supérieure de la tige 40 et le côté inférieur 21 B du composant astragalien 20 sont envisageables, tant que ces moyens conduisent à la mise en prise de l'extrémité inférieure de la tige avec le calcanéum C pour comprimer ce dernier contre l'astragale A ; ainsi, un accouplement par une structure à baïonnette est possible, tout comme des structures d'accouplement à solliciter exclusivement par translation rectiligne ;
- grâce à leur appui glissant plan/plan au niveau de leur surface 11A et 30A, le composant tibial 10 et le patin 30 sont mobiles l'un par rapport à l'autre, ce qui conduit généralement à désigner la prothèse de cheville constituée des éléments 10, 20 et 30, de prothèse « à patin mobile » ; cependant, en variante, le composant astragalien 20 peut être associé à un patin dit « fixe », c'est-à-dire à un patin rapporté de manière fixe contre un composant tibial ;
- le composant astragalien 20 et la tige astragalo-calcanéenne 40 ne sont pas nécessairement à utiliser conjointement avec un composant tibial et un patin prothétique tels que les éléments 10 et 30 ; le composant astragalien peut en effet être prévu pour coopérer de manière articulée directement avec l'extrémité inférieure anatomique du tibia T si cette dernière présente des capacités articulaires suffisantes ; l'ensemble prothétique de cheville peut alors être qualifié d'ensemble prothétique partiel, tandis que l'ensemble prothétique 1 de la figure 1 est à considérer comme un ensemble prothétique total ; et/ou
- si besoin, la fixation du composant astragalien 20 sur l'astragale A peut être renforcée par des moyens additionnels.

## Revendications

1. Ensemble prothétique de cheville (1), comportant, d'une part, un composant astragalien (20) d'une prothèse articulaire de cheville, adapté pour être fixé sur l'astragale (A) d'un patient et, d'autre part, une tige astragalo-calcanéenne (40) dont une première extrémité longitudinale (41) est adaptée pour être fixée au composant astragalien, par complémentarité de formes avec une partie dédiée (22A) du composant astragalien, qui est prévue sur un côté inférieur (21 B) de ce composant, destiné à être tourné vers une face supérieure (A₁) de l'astragale,
**caractérisé en ce que** la tige astragalo-calcanéenne (40) est munie, à sa seconde extrémité longitudinale (43), d'un moyen (43A ; 45) de mise en prise osseuse du calcanéum (C) du patient, adapté pour rapprocher et ainsi comprimer le calcanéum contre l'astragale (A) lors de la fixation de la première extrémité (41) de la tige à la partie dédiée (22A) du composant astragalien (20).

2. Ensemble prothétique suivant la revendication 1, **caractérisé en ce que** la tige astragalo-calcanéenne (40) est adaptée pour s'étendre en longueur depuis la face supérieure (A₁) de l'astragale (A) sur laquelle le composant astragalien (20) est fixé, jusqu'à une face inférieure (Ci) du calcanéum (C) opposée à l'astragale.

3. Ensemble prothétique suivant l'une des revendications 1 ou 2, **caractérisé en ce que** le moyen de mise en prise comprend un filetage (43A), adapté pour s'engager dans la matière osseuse du calcanéum (C) lors de la fixation de la première extrémité (41) de la tige astragalo-calcanéenne (40) dans la partie dédiée (22A) du composant astragalien (20).

4. Ensemble prothétique suivant l'une des revendications 1 ou 2, **caractérisé en ce que** le moyen de mise en prise comprend un anneau (45), entourant la seconde extrémité (43) de la tige astragalo-calcanéenne (40) de manière fixe ou mobile et adapté pour s'appuyer contre une face inférieure (C₁) du calcanéum (C), opposée à l'astragale (A), lors de la fixation de la première extrémité (41) de la tige dans la partie dédiée (22A) du composant astragalien (20).

5. Ensemble prothétique suivant l'une quelconque des revendications précédentes, **caractérisé en ce que** le moyen (43A ; 45) de mise en prise du calcanéum (C) présente une dimension transversale maximale (d₄₃) strictement supérieure à la dimension transversale maximale (d₄₁) d'un moyen (41A) de fixation de la première extrémité (41) de la tige astragalo-calcanéenne (40) au composant astragalien (20).

6. Ensemble prothétique suivant l'une quelconque des revendications précédentes, **caractérisé en ce que** l'une parmi la première extrémité (41) de la tige astragalo-calcanéenne (40) et la partie dédiée (22A) du composant astragalien (20) est munie d'un filetage (41A), tandis que l'autre forme un taraudage (22A) sensiblement complémentaire.

7. Ensemble prothétique suivant l'une quelconque des revendications précédentes, **caractérisé en ce que** le composant astragalien (20) est muni, sur son côté inférieur (21 B), d'un moyen (22) d'ancrage osseux dans l'astragale (A), à l'intérieur duquel est agencée ladite partie dédiée (22A).

8. Ensemble prothétique suivant les revendications 6 et 7 prises ensemble, **caractérisé en ce que** le moyen d'ancrage inclut un plot creux (22) dans lequel le filetage ou le taraudage (22A) du composant astragalien (20) est délimité, de préférence de manière sensiblement co-axiale au reste du plot.

9. Ensemble prothétique suivant l'une quelconque des revendications précédentes, **caractérisé en ce que** la tige astragalo-calcanéenne (40) est munie, à sa seconde extrémité longitudinale (43), d'un profil (44A) d'entraînement, notamment d'entraînement en rotation sur elle-même, adapté pour coopérer avec un outil (61) d'accouplement de la tige avec le composant astragalien (20).

10. Ensemble prothétique suivant la revendication 9, **caractérisé en ce que** ledit profil (44A) ou un autre profil d'entraînement de la tige (40) est adapté pour coopérer avec un outil de désaccouplement entre la tige et le composant astragalien (20).

11. Ensemble prothétique suivant l'une quelconque des revendications précédentes, **caractérisé en ce qu'**il comprend en outre, à la fois, un composant tibial (10) d'une prothèse articulaire de cheville, adapté pour être fixé sur le tibia (T) du patient, et un patin prothétique (30) adapté pour être interposé entre les composants astragalien (20) et tibial (10).
